# EUROPEAN PATENT APPLICATION

(11) **EP 4 585 903 A2**
(43) Date of publication of application: **16.07.2025**
(21) Application number: 25173914.0
(22) Date of filing: 25.02.2019
(51) Int. Cl.: G01N 15/10

(54) **TISSUE ANALYSIS BY MASS SPECTROMETRY**

(30) Priority: 23.02.2018 US 201862634561 P; 08.03.2018 US 201862640550 P
(62) Divisional of application: 19757574.9
(71) Applicant: Board of Regents, The University of Texas System, Austin, TX 78701 (US); Baylor College of Medicine, Houston, TX 77030 (US)
(72) Inventor: The designation of the inventor has not yet been filed
(74) Representative: Vossius & Partner Patentanwälte Rechtsanwälte mbB

(57) **Abstract**

Methods and devices are provided for assessing biological samples using molecular analysis. In certain aspects, methods and devices of the embodiments allow for the collection of liquid tissue samples and delivery of the samples for mass spectrometry. In certain aspects, the results of the mass spectrometry can be analyzed to determine tissue type, sample quality, or disease state of the sample.

## Description

This application claims the benefit of United States Provisional Patent Application Nos. 62/634,561, filed February 23, 2018, and 62/640,550, filed March 8, 2018, each of which is incorporated herein by reference in their entirety.

This invention was made with government support under Grant no. R00 CA190783 awarded by the National Institutes of Health. The government has certain rights in the invention.

### BACKGROUND OF THE INVENTION

### 1. Field of the Invention

The present invention relates generally to the field of medicine, molecular biology and biochemistry. More particularly, it concerns methods and devices for assessment of tissue samples using mass spectrometry.

### 2. Description of Related Art

Clinical diagnosis is commonly performed through the evaluation of tissue samples pre-operatively, intra-operative, and post-operatively, at several other stages of the patient's treatment process. Tissue evaluation is very critical in the diagnosis and management of cancer patients. Intra-operative pathologic assessment of excised tissues, for example, is routinely performed for diagnosis and surgical margin evaluation in a variety of cancer surgeries. The resected tissue specimens are sent to a nearby room, often called the "frozen room", for tissue preparation, staining, and evaluation. The tissue specimen is frozen, sectioned, stained, and interrogated using light microscopy by an expert pathologist who carefully evaluates if the surgical margins contain cancer cells (positive margin) or not (negative margin). While intraoperative frozen section analysis has been performed in clinical practice for decades, it presents many challenges. Freezing artifacts occur during tissue processing and interfere with tissue structure and cell morphology, thus complicating pathologic interpretation. Moreover, certain tumor cells are very difficult to recognize due to their atypical pattern of growth and shape. Expert surgeons have difficulty in determining malignancy of thyroid nodules and lymph nodes and a similar problem exists for distinguishing parathyroid tissue from lymph node tissue, thymic tissue, and thyroid tissue. Re-operative cervical surgery following failed resection of thyroid or parathyroid tissue is extremely costly with an associated 10-fold higher complication rate and significant decrease in quality of life to the patient. Molecular approaches could provide highly accurate and potentially real-time assessments of tissue samples, and possibly prevent unnecessary invasive surgical procedures.

### SUMMARY OF THE INVENTION

In some embodiments the present disclosure provides a method for assessing tissue samples from a subject during cervical surgery comprising applying a fixed or discrete volume of a solvent to a tissue site in the subject, collecting the applied solvent to obtain a liquid sample, subjecting the sample to mass spectrometry analysis, and identifying the tissue site as thyroid tissue, thymic tissue, parathyroid tissue or lymph node tissue (e.g., cervical lymph node tissue such as tissue from a lateral jugular, anterior jugular, or jugulo-digastric lymph node). In some aspects, the method further comprises surgically removing a portion of the tissue based on said identifying. In some aspects, the portion of tissue is thyroid tissue. In some aspects, the method further comprises identifying the tissue site as cancer or non-cancer tissue.

In certain aspects a method of the embodiments is automated (e.g., performed by a robot). For example, in some cases, one of more of the following steps is automated (a) applying a fixed or discrete volume of a solvent to a tissue site in the subject; (b) collecting the applied solvent to obtain a liquid sample; (c) subjecting the sample to mass spectrometry analysis; and/or (d) identifying the tissue site as thyroid tissue, thymic tissue, parathyroid tissue or lymph node tissue. In preferred aspects, the identifying is of tissue as thyroid tissue, thymic tissue, parathyroid tissue, lymph node tissue or as cancerous or non-cancerous is completed by a computer. In further aspects, an identified tissue is surgically removed and in some case this surgery is automated. In further aspects, the identifying is completed by a computer and the computer then directs the surgical removal of a selected tissue (by a robotic surgical device).

In some aspects, the fixed or discrete volume of a solvent is not applied as a spray. In some aspects, the fixed or discrete volume of a solvent is applied as a droplet. In certain aspects, the fixed or discrete volume of a solvent is applied at using a pressure of less than 100 psig. In particular aspects, the fixed or discrete volume of a solvent is applied at using a pressure of less than 10 psig. In some aspects, the fixed or discrete volume of a solvent is applied at using a mechanical pump to move the solvent through a solvent conduit. In some aspects, collecting the applied solvent comprises applying a negative pressure to pull the sample into a collection conduit and/or applying a gas pressure to push the sample into a collection conduit. In certain aspects, collecting the applied solvent comprises applying a negative pressure to pull the sample into a collection conduit and applying a positive pressure to push the sample into a collection conduit. In some aspects, the solvent is applied through a solvent conduit that is separate from the collection conduit. In certain aspects, the gas pressure is applied through a gas conduit that is separate from the solvent conduit and the collection conduit. In some aspects, applying a gas pressure to push the sample into a collection conduit comprises applying a pressure of less than 100 psig.

In some aspects, the methods of the embodiments produce no detectable physical damage to the tissue site. In some aspects, the method does not involve application of ultrasonic or vibrational energy to the tissue. In some aspects, the solvent is sterile. In some aspects, the solvent is pharmaceutically acceptable formulation. In certain aspects, the solvent is an aqueous solution. In particular aspects, the solvent is sterile water. In some aspects, the solvent consists essentially of water. In some aspects, the solvent comprises from about 1 to 20% of an alcohol. In certain aspects, the alcohol comprises ethanol.

In some aspects, the discrete volume of solvent is between about 0.1 and 100 µL. In particular aspects, the discrete volume of solvent is between about 1 and 50 µL. In some aspects, collecting the applied solvent is between 0.1 and 30 seconds after the applying step. In some aspects, collecting the applied solvent is between 1 and 10 seconds after the applying step. In some aspects, the tissue site is an internal tissue site *(e.g.,* in a living tissue) that is being surgically assessed. In some aspects, the liquid samples are collected with a probe. In particular aspects, the probe is washed between collection of the different samples. In certain aspects, the probe comprises a disposable portion that contacts the tissue site and where said disposable portion is changed between collection of the different samples. In some aspects, the probe comprises a collection tip and further comprising ejecting the collection tip from the probe after the liquid samples are collected. sed.

In further aspects, the method comprises collecting a plurality liquid samples from a plurality of tissue sites. In some aspects, the plurality of tissue sites comprises 2, 3, 4, 5, 6, 7, 8, 9 or 10 tissues sites. In some aspects, the plurality of tissue sites surrounds a section of tissue that has been surgically resected. In some aspects, the resected tissue is a tumor.

In further aspects, the method may be defined as an intraoperative method. In some aspects, the mass spectrometry comprises ambient ionization MS. In some aspects, subjecting the sample to mass spectrometry analysis comprises determining a profile corresponding to the tissue site. In some aspects, methods of the embodiments further comprise comparing the profile to a reference profile to identify tissue sites that include diseased tissue. In some aspects, the methods further comprise resecting tissue sites that are identified to include diseased tissue.

Still a further embodiment provides an apparatus for obtaining samples (*e.g*., from thyroid, thymic, parathyroid or lymph node tissues, such as during cervical surgery) for mass spectrometry analysis, the apparatus comprising: a chamber comprising a solvent; a pressurized gas supply; a mass spectrometer; a probe comprising a reservoir, a first conduit, a second conduit and a third conduit, wherein: the reservoir is in fluid communication with the first conduit, the second conduit and the third conduit; the first (solvent) conduit is in fluid communication with the chamber; the second (gas) conduit is in fluid communication with pressurized gas supply; and the third (collection) conduit is in fluid communication with the mass spectrometer. In further aspects, the mass spectrometer in communication with a computer that provides a sample analysis. In certain aspects, the results of each sample analysis are provided by a visual or auditory output from the computer. For example, the results of each sample analysis by the computer can be indicated by a differently colored light that is illuminated or by a different frequency of sound produced. In some aspects, the mass spectrometer is a mobile the mass spectrometer. In further aspects, the mass spectrometer can comprise an uninterruptable power supply *(e.g.,* a battery power supply). In still further aspects, the mass spectrometer comprises an inlet that may be closed to keep instrument vacuum. In yet further aspects, the mass spectrometer is separated from the probe by a mesh filter *(e.g.,* to block contamination). Thus, in some aspects, there is provided an apparatus of the embodiments that comprises a sample collected from a parathyroid tissue or cervical lymph node *(e.g.,* lateral jugular, anterior jugular, or jugulo-digastric lymph node).

In some aspects, the reservoir is configured to form a droplet of the solvent. In certain aspects, the pressurized gas supply provides a gas to the probe at a pressure between 0.1 psig and 5.0 psig. In further aspects, the pressurized gas supply provides a gas to the probe at a pressure between 0.5 psig and 2.5 psig. In several aspects, the pressurized gas supply provides air to the probe. In other aspects, the pressurized gas supply provides an inert gas such as nitrogen or carbon dioxide to the probe.

In additional aspects, the apparatus further comprises a pump configured to transfer the solvent from the chamber to the first conduit. In further aspects, the apparatus may comprise a first valve configured to control a flow from the third conduit to the mass spectrometer. In some aspects, the third conduit is under a vacuum when the first valve is in the open position. In other aspects, the apparatus may comprise a second valve configured to control a flow of pressurized gas through the second conduit.

In certain aspects, the solvent may comprise water and/or ethanol. In several aspects, the probe is formed from polydimethylsiloxane (PDMS) and/or polytetrafluoroethylene (PTFE). In some aspects, the probe is disposable. In particular aspects, the probe may include a collection tip that is ejectable (*e.g*. capable of being ejected from the probe). In further aspects, the probe comprises a tracking device configured to track a location of the probe. In some aspects, the reservoir has a volume between 1 microliter and 500 microliters, between about 1 microliter and 100 microliters or between about 2 microliters and 50 microliters. In additional aspects, the reservoir has a volume between 5.0 microliters and 20 microliters.

In still further aspects, the apparatus may additionally comprise a control system configured to control: a solvent flow *(e.g.,* flow of a fixed or discrete volume of solvent) from the chamber through the first conduit to the reservoir; a pressurized gas flow from the pressurized gas supply through the second conduit to the reservoir; and a sample flow from the reservoir through the third conduit to the mass spectrometer. In some aspects, the control system is configured to: control the solvent flow at a flow rate between 100 and 5000 microliters per minute (*e.g*., between 200 and 400 microliters per minute) for a period of time between 1 and 3 seconds; control the pressurized gas flow at a flow rate between 1 and 10 psig for a period of time between 10 and 15 seconds; and control the sample flow for a period of time between 10 and 15 seconds. For example, in some aspects, the control system comprises a trigger or button to initiate solvent flow. In further aspects, the control system comprises a pedal (*i.e.,* that can be operated by foot action) to initiate solvent flow. A skilled artisan will recognize that the lengths of the first and/or second conduit may be adjusted to fit the particular use of the system. In yet further aspects, the control system is configured to control: a solvent flow (*e.g*., flow rate for a fixed period of time) from the chamber through the first conduit to the reservoir. In further aspects, an apparatus of the embodiments does not include a device for producing ultrasonic or vibrational energy (*e.g.,* in sufficient amounts to disrupt tissues). Devices and methods, for noninvasive MS analysis of samples that may be used according to the embodiments are provided in U.S. Patent Application No. 15/692,167 and in Zhang *et al*., 2017 each of which is incorporated herein by reference in its entirety.

In some embodiments, the present disclosure provides an assay method comprising obtaining a fine needle aspirate biopsy sample from a subject and performing mass spectrometry (MS) on the sample. In some aspects, fine needle aspirate biopsy is from an organ or suspected tumor. In preferred aspects, the fine needle aspirate biopsy is from thyroid tissue (*e.g.,* of a thyroid nodule). In some aspects, the assay method is further defined as an *ex vivo* method. In some aspects, the method is further defined as a method for detecting a cancer, such as thyroid cancer, in the subject. In certain aspects, a method of the embodiment is further defined as a method for detecting (or identifying) if the thyroid nodule from which the fine-needle aspirate biopsy was obtained is benign or malignant. In some aspects, the thyroid cancer is oncocytic thyroid cancer. In certain aspects, the thyroid cancer is papillary thyroid cancer, follicular thyroid cancer, anaplastic thyroid cancer, or medullary thyroid cancer. In some aspects, a method involves identifying is a sample is from tissue that is a papillary thyroid cancer vs benign tissue; follicular thyroid cancer vs benign tissue; anaplastic thyroid cancer vs benign tissue; or medullary thyroid cancer vs benign tissue. In certain aspects, the benign tissue is papillary thyroid adenoma or normal thyroid tissue. In some aspects, the ambient ionization MS comprises DESI-MSI imaging. In some specific aspects, a method comprises expelling the fine-needle aspirate biopsy sample onto a glass slide, and imaging the slide by DESI-MS in the region with FNA material. In some aspects, performing ambient ionization comprises measuring a level of a lipids and/or metabolites in the sample. Methods, for MS analysis of samples that may be used according to the embodiments are provided in U.S. Patent Application No. 15/648,276, which is incorporated herein by reference in its entirety.

In further aspects, the method comprises obtaining a reference profile and detecting the presence of cancer cells by comparing the profile from the sample to a reference profile. In some aspects, the reference profile is obtained from the same subject. In some aspects, the reference profile is obtained from a different subject or a combination of different subjects. In further aspects, the method may further comprise administering at least a first anticancer therapy to a subject identified to have a cancer. In some aspects, the anticancer therapy comprises radiation, immunotherapy, surgery or chemotherapy therapy. In some aspects, the cancer is a thyroid cancer and the anticancer therapy is an iodine based therapy.

In further aspects, a method for analyzing a FNA sample of the embodiments comprises using a statistical classifier built from the lipid and/or metabolic information obtained from reference profiles by MS methods to identify if a thyroid sample obtained from a nodule is benign or malignant. For example, the statistical classifier can be built from reference profiles obtained from tissue samples by MS methods or built from reference profiles obtained from fine-needle biopsy samples by MS methods. In further aspects, the statistical classifier is built from reference profiles obtained from a combination of tissue samples and fine-needle biopsy samples by MS methods. In certain aspects, the statistical classifier is built as a two-class classifier that can identify nodules as benign thyroid adenomas or malignant thyroid carcinomas; as benign thyroid, including adenomas and normal thyroid, or malignant thyroid carcinomas. In further aspects, the statistical classifier is built as a three-class classifier that can identify nodules as benign thyroid, thyroid adenomas, or thyroid carcinomas. Thus, in some aspects, samples are analyzed from a thyroid tumor that is a follicular neoplasm, a papillary neoplasm or a medullary neoplasm. In some aspects, the classifier predicts using the data acquired from one or more pixels of the FNA in the glass slide, such as when the one or more pixels that contain one or more cells. In further aspects, the FNA classification result is based on a single pixel result or a combination of the predictions given to each individual pixel. In certain aspects, the cutoff value for sample classification is optimized based on results for FNA samples. In yet further aspects of the embodiments, a FNA sample is subjected to histology after MS analysis. Thus, in some aspects, one or more pixels with one or more cells are identified in the FNA sample by an auxiliary technique after MS analysis. For example, the auxiliary technique can be pathology or a spectroscopic method. In further aspects, the MS result obtained by a method of the embodiments is combined to genetic classifier result to obtain a final diagnosis for the FNA analyzed. In certain aspects, a prediction based on MS is made when cell counts are sufficient; when reaching probability cut-off of a pixel; or when reaching a cut-off of all pixels from one FNA sample.

As used herein a "fine-needle aspirate" refers to a sample taken from a tissue site using a needle to extract the same. In preferred aspects, fine-needle aspirate sample refers to a sample collected with a needle smaller than 19 gauge, such as a 20 or 22 gauge needle. In some aspects, a fine-needle aspirate refers to a substantially liquid sample.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is preferably below 0.01%. Most preferred is a composition in which no amount of the specified component can be detected with standard analytical methods.

As used herein in the specification and claims, "a" or "an" may mean one or more. As used herein in the specification and claims, when used in conjunction with the word "comprising", the words "a" or "an" may mean one or more than one. As used herein, in the specification and claim, "another" or "a further" may mean at least a second or more.

As used herein in the specification and claims, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating certain embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****: Schematic of the DESI-MS LASSO method.**
**FIG. 2****: Representative Stained and Negative Ion DESI-MS Imaged Tissue Sections.** Normal thyroid, follicular thyroid adenoma (FTA), and follicular thyroid carcinoma (FTC) tissue sections were imaged by DESI-MS in negative ion mode. Images are shown for representative selected *m*/*z* values corresponding to specific lipids. Following DESI-MS imaging, tissues were H&E stained and evaluated by a pathologist.
**FIG. 3****: Representative Mass Spectra of Tissue Section Negative Ion DESI-MS.** Shown are the Negative Ion DESI-MS spectra of the normal, FTA, and FTC tissue sections shown in FIG. 2.
**FIG. 4****: Representative Stained and Negative Ion DESI-MS Imaged Tissue Sections.** Non oncocytic, and oncocytic FTA and FTC tissue sections were imaged by DESI-MS in negative ion mode. Images are shown for selected *m*/*z* values corresponding to specific lipids. Following DESI-MS imaging, tissues were H&E stained and evaluated by a pathologist. Follicular lesions are outlined in black.
**FIG. 5****: Representative Mass Spectra of Negative Ion DESI-MS Imaged Tissue Sections.** Shown are representative Negative Ion DESI-MS spectra of FTA and FTC sections from FIG. 4.
**FIG. 6****: Representative Stained and Positive ION DESI-MS Imaged Tissue Sections.** Normal thyroid, follicular thyroid adenoma (FTA), and follicular thyroid carcinoma (FTC) tissue sections were imaged by DESI-MS in positive ion mode. Images are shown for representative selected *m*/*z* values corresponding to specific lipids. Following DESI-MS imaging, tissues were H&E stained and evaluated by a pathologist.
**FIG. 7****: Representative Mass Spectra of Positive Ion DESI-MS Imaged Tissue Sections.** Shown are the Positive Ion DESI-MS spectra of the normal, FTA, and FTC sections from FIG. 6.
**FIG. 8****: Representative Stained and Positive ION DESI-MS Imaged Tissue Sections.** Non oncocytic, and oncocytic FTA and FTC tissue sections were imaged by DESI-MS in positive ion mode. Images are shown for selected *m*/*z* values corresponding to specific lipids. Following DESI-MS imaging, tissues were H&E stained and evaluated by a pathologist. Follicular lesions are outlined in black.
**FIG. 9****: Representative Mass Spectra of Positive Ion DESI-MS Imaged Tissue Sections.** Shown are representative Positive Ion DESI-MS spectra of FTA and FTC sections from FIG. 8.
**FIG. 10****: LASSO Weights of MS Peaks Found in Negative Ion DESI-MS.** LASSO weights were determined by statistical analysis and assigned based on features specific to tissue type.
**FIG. 11****: LASSO Weights of MS Peaks Found in Positive Ion DESI-MS.** LASSO weights were determined by statistical analysis and assigned based on features specific to tissue type.
**FIG. 12****: LASSO Weights of MS Peaks identified in Negative Ion DESI-MS.** Shown are LASSO weights generated by comparison between only FTA and FTC tissues, and the prediction accuracy of LASSO results by pixel and patient.
**FIG. 13****: Attribution of Selected Ion Species Contributing to LASSO Classification.** Shown are the *m*/*z* peak, tentative attribution, and weight in LASSO classification.
**FIG. 14****: DESI-MS Spectra of a Thyroid Fine Needle Aspirate Sample.** Shown are Negative Ion DESI-MS and Positive Ion DESI-MS for a thyroid fine needle aspirate sample.
**FIG. 15****: Negative Ion DESI-MS Spectrum of a Thyroid Fine Needle Aspirate Sample.** Shown is the Negative Ion DESI-MS spectrum for a second thyroid fine needle aspirate sample.

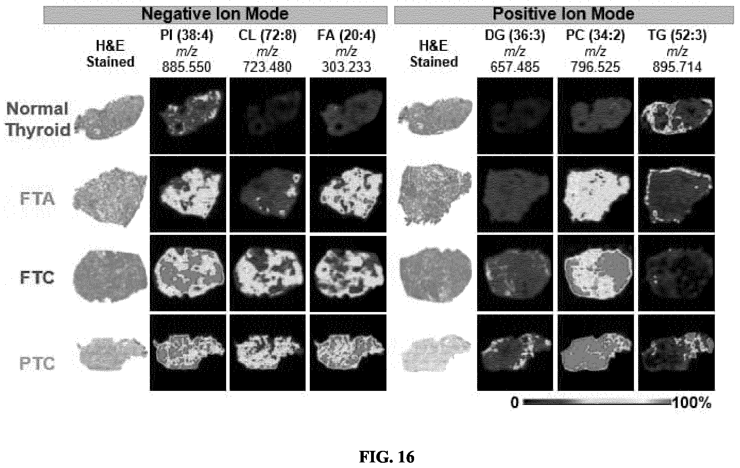

**FIG. 16****:** Figure depicts analysis of thyroid tissues by DESI-MS in the negative and positive ion mode. Ion images of normal thyroid, FTA, FTC, and PTC. X:Y represents the different number of carbon atoms and the different number of double bonds in the fatty acid chains.

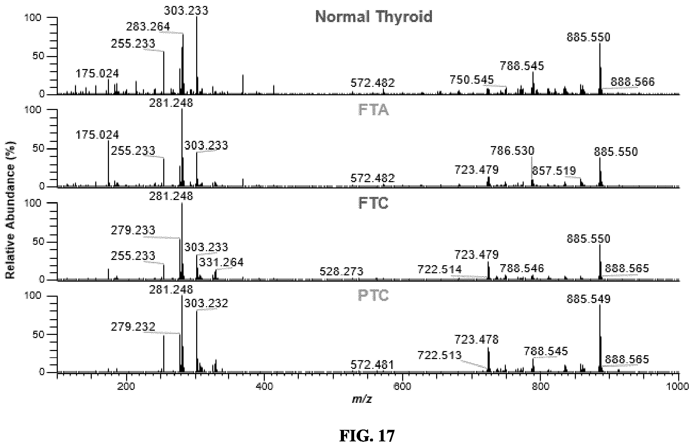

**FIG. 17****:** A representative analysis of thyroid tissues by DESI-MS in the negative ion mode. Representative metabolic profiles of normal thyroid, FTA, FTC, and PTC.

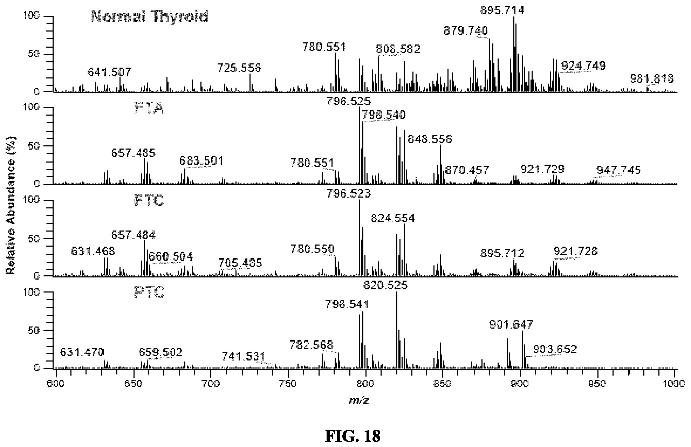

**FIG. 18****:** A representative analysis of thyroid tissues by DESI-MS in the positive ion mode. Representative metabolic profiles of normal thyroid, FTA, FTC, and PTC.

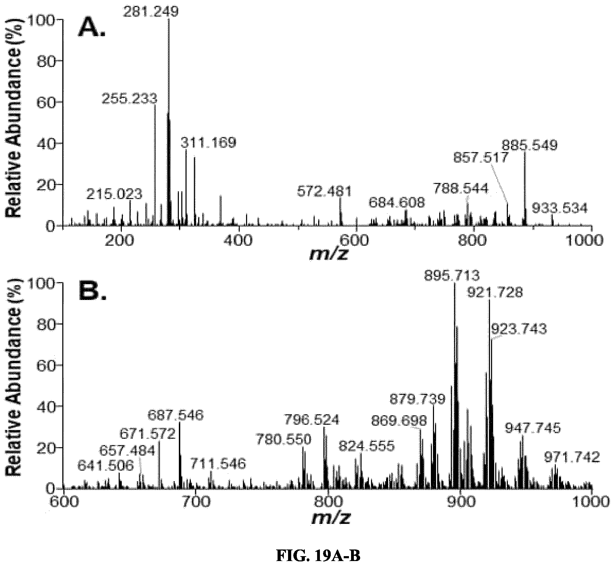

**FIG. 19A****-B:** Representative DESI-MS spectra obtained from a clinical fine needle aspiration sample of a normal thyroid sample in the negative (A) and positive (B) ion modes.
**FIG. 20****:** The table shows lasso predictions per-pixel and per patient for the benign vs. PTC and benign vs. FTC models. Rows indicate the diagnosis determined by pathology and columns indicate the diagnosis predicted by the lasso model. Class accuracies and overall accuracies are defined as agreement between the diagnosis predicted by lasso and the true diagnosis determined by pathology.
**FIG. 21****:** Table shows per-sample Lasso predictions on clinical FNA mass spectra. The top two models show the prediction of the benign vs. PTC and benign vs. FTC models generated from tissue data on clinical FNA data. The bottom model shows the crossvalidation prediction for the benign vs. PTC model generated from clinical FNA data. Rows indicate the diagnosis determined by pathology and columns indicate the diagnosis predicted by the lasso model. Overall accuracies are defined as agreement between the diagnosis predicted by lasso and the diagnosis determined by pathology.

### DESCRIPTION OF ILLUSTRATIVE EMBODIMENTS

### I. The Present Embodiments

In certain aspects, the instant application provides methods and devices for molecular assessment of samples, such as tissue samples and fine needle aspirates. In particular, aspects the methods can be used to assess multiple tissue sites during an operation (or biopsy) of the tissue. This feature allows for accurate identification of diseased tissues (e.g., tissue sites retaining cancer cells) in "real-time" allowing surgeons to more accurately address only the diseased tissue relative to surrounding normal tissues. In particular aspects, the methods disclosed here can involve delivery of a fixed or discrete volume of solvent to a tissue site, followed by collection of a liquid sample from the site and analysis of the liquid sample by mass spectrometry. For example, it has been demonstrated that such non-invasive MS analysis techniques can accurately identify thyroid vs. thymic vs. parathyroid vs. lymph node tissue to guide surgical intervention. Importantly, rather than being applied in a high pressure spray, solvent used for tissue analysis is applied as discreet droplets and at low pressure. These methods allow for accurate collection of samples from a distinct tissue site while avoiding damage to the tissue being assessed. The resulting mass spectrometry profile from collected samples allows for differentiation of diseased versus normal tissue sites. The method can be repeated at multiple sites of interest to very accurately map molecular changes (*e.g.,* in a tissue). The method can also be performed *ex vivo,* such as for the analysis of fine needle aspirates. Importantly, the profiles of samples could be differentiated even with-out the use an ionization source. Thus, while methods of the embodiments could be used in conjunction with an ionization source, the use of such a source is not required. These methodologies can allow assessment of plurality of tissue sites over a short range of time, thereby allowing for very accurate assessment of the boundaries of diseased versus normal tissues.

In some aspects, the materials (PDMS and PTFE) and solvent (*e.g*., water only solvents) used in the devices of the embodiments are biologically compatible, such that they can be used in surgery in for real-time analysis. Furthermore, because the devices can be very compact, it can be hand-held or integrated to a robotic surgical system, such as the Da Vinci surgical system (*e.g*., in an automated system). Thus, many regions of the human body cavity can be quickly sampled during surgery, and analyzed (*e.g*., by using a database of molecular signatures and machine learning algorithms). Therefore, the diagnostic results may be provided in real time for each sampled region.

In some aspects, methods are and devices described herein for detecting cancer cells and, in particular, oncocytic tumor cells using mass spectrometry and statistical analysis using LASSO. In particular, molecular profiles can be generated from the MS data and the LASSO statistical method. In the studies herein, DESI-MS was used to image and chemically characterize the lipid composition of a variety of thyroid and parathyroid tissues. The analysis revealed a novel method for generating molecular signatures to differentiate between tissue types and disease states. DESI-MS imaging and IHC experiments confirmed that the spatial distribution of the analyzed molecular ions accurately reflected a pathologists diagnosis.

In some aspects, methods and devices are described for tissue identification during cervical surgery. These methods and devices may be been applied to characterize thyroid, thymic, parathyroid and lymph node samples, with the goal of using it in surgery to improve precision, reduce need for frozen section analysis and improve outcomes for patients. Using an initial sample set of 72 tissue samples, it is shown that the MasSpec Pen is over 93% accurate in discriminating normal thyroid, normal thymic, normal parathyroid or normal lymph node tissues based on molecular information. In a subsequent study with a set of normal, follicular thyroid adenoma, and follicular thyroid carcinoma samples, it is shown that the MasSpec Pan can effectively distinguish between each, with accuracy over 94%. These results indicate that the MasSpec Pen can be used to accurately distinguish between tissue types and disease states.

Finally, methods are described for the analysis of fine needle aspirate samples by mass spectrometry and the LASSO statistical method. It is shown that the methods presented herein can generate rich MS spectra from fine needle aspirate samples, which may be used to prevent unnecessary surgical procedures in the future.

### II. Surgical methods

In some aspects, there are provided surgical methods, such as for cervical surgery. In preferred aspects these methods may be laparoscopic surgical methods, which may be performed manually or by a robotic surgical device. For example, in some aspects an autonomous Mass Spec Image Guided method is provided. An exemplary autonomous surgical method may comprise:
1. Prior to surgery a patient will have high resolution imaging scan performed on the area to be operated on. For example, the imaging scan can be a CT or MRI of the tissue site(s).
2. Optionally, fiducials will then be placed on the patient to allow for 3D dynamic spatial resolution of the images of the tissue site.
3. Image guided surgery will commence by the robot. In some aspects, some or all of the surgical instruments will have geospatial recognition tags so that the exact position of each tip of instrument is known in relationship to the preoperative imaging where geospatial recognition tags will correspond to a position in three-dimensional reconstruction images from the prior imaging. For example, CT scan data or MRI DICOM files may be used.
4. As tissue layers are opened, a mass spec pen/probe of the embodiments will scan the tissue being dissected to verify that the tissue being worked on is indeed the tissue layer represented on the CT scan (*e.g.,* to confirm that a tissue is thyroid, thymic, parathyroid or lymph). In this way the dissection will proceed *via* a image targeted approach with confirmation of layer by layer of tissue dissection using the mass spec pen.
5. Optionally, a dual input logic controller will be used to control dissection in a closed loop manner. As tissue is dissected and simultaneously scanned if the mass spec pen correlates with tissue layer and target as determined by 3D imaging then the dissection will continue to proceed. However if tissue is not able to be verified than the surgeon will need to verify before allowing the robot to proceed further.

### III. Assay Methodologies

In some aspects, the present disclosure provides methods of determining the presence of diseased tissue (*e.g*., tumor tissue) or detecting a molecular signature of a biological specimen by identifying specific patterns of a mass spectrometry profile. Biological specimens for analysis can be from animals, plants or any material (living or non-living) that has been in contact with biological molecules or organisms. A biological specimen can be sampled *in vivo* or *ex vivo. In vivo* detection can be performed intrasurgically, such as during a routine surgical procedure or during a laparoscopic or endoscopic surgical procedure. *Ex vivo* procedures described herein can be used, *e.g.,* to analyze fine-needle aspirate sample.

A profile obtained by the methods of the embodiments can correspond to, for example, proteins, metabolites, or lipids from analyzed biological specimens or tissue sites. These patterns may be determined by measuring the presence of specific ions using mass spectrometry. Some non-limiting examples of ionizations methods that can be coupled to this device include chemical ionization, laser ionization, atmospheric-pressure chemical ionization, electron ionization, fast atom bombardment, electrospray ionization, thermal ionization. Additional ionization methods include inductively coupled plasma sources, photoionization, glow discharge, field desorption, thermospray, desorption/ionization on silicon, direct analysis in real time, secondary ion mass spectroscopy, spark ionization, and thermal ionization.

In particular, the present methods may be applied or coupled to an ambient ionization source or method for obtaining the mass spectral data such as extraction ambient ionization source. Extraction ambient ionization sources are methods with, in this case, liquid extraction processes dynamically followed by ionization. Some non-limiting examples of extraction ambient ionization sources include air flow-assisted desorption electrospray ionization (AFADESI), direct analysis in real time (DART), desorption electrospray ionization (DESI), desorption ionization by charge exchange (DICE), electrode-assisted desorption electrospray ionization (EADESI), electrospray laser desorption ionization (ELDI), electrostatic spray ionization (ESTASI), Jet desorption electrospray ionization (JeDI), laser assisted desorption electrospray ionization (LADESI), laser desorption electrospray ionization (LDESI), matrix-assisted laser desorption electrospray ionization (MALDESI), nanospray desorption electrospray ionization (nano-DESI), or transmission mode desorption electrospray ionization (TM-DESI). In certain aspects, DESI mass spectrometry is used to assess biological samples.

DESI is an ionization technique used to prepare a mass spectra of organic molecules or biomolecules. The ionization technique is an ambient ionization technique which uses atmospheric pressure in the open air and under ambient conditions. DESI is an ionization technique which combines two other ionization techniques: electrospray ionization as well as desorption ionization. Ionization is affected by directing electrically charged droplets at the surface that is millimeters away from the electrospray source. The electrospray mist is then pneumatically directed at the sample. Resultant droplets are desorbed and collected by the inlet into the mass spectrometer. These resultant droplets contain additional analytes which have been desorbed and ionized from the surface. These analytes travel through the air at atmospheric pressure into the mass spectrometer for determination of mass and charge. One of the hallmarks of DESI is the ability to achieve ambient ionization without substantial sample preparation.

As with many mass spectrometry methods, ionization efficiency can be optimized by modifying the collection or solvent conditions such as the solvent components, the pH, the gas flow rates, the applied voltage, and other aspects which affect ionization of the sample solution. In particular, the present methods contemplate the use of a solvent or solution which is compatible with human issue. Some non-limiting examples of solvent which may be used as the ionization solvent include water, ethanol, methanol, acetonitrile, dimethylformamide, an acid, or a mixture thereof. In some embodiments, the method contemplates a mixture of acetonitrile and dimethylformamide. The amounts of acetonitrile and dimethylformamide may be varied to enhance the extraction of the analytes from the sample as well as increase the ionization and volatility of the sample. In some embodiments, the composition contains from about 5:1 (v/v) dimethylformamide:acetonitrile to about 1:5 (v/v) dimethylformamide:acetonitrile such as 1: 1 (v/v) dimethylformamide:acetonitrile. In some embodiments, the solvent is acetonitrile. However, in preferred embodiment the solvent for use according to the embodiments is a pharmaceutically acceptable solvent, such as sterile water or a buffered aqueous solution.

Additionally, two useful parameters are the impact angle of the spray and the distance from the spray tip to the surface. Generally, the electrospray tip is placed from about 0.1-25 mm from the surface especially from 1-10 mm. In some embodiments, a placement from about 3-8 mm is useful for a wide range of different application such as those described herein. Additionally, varying the angle of the tip to the surface (known as the incident angle or α) may be used to optimize the ionization efficacy. In some embodiments, the incident angle may be from about 0° to about 90°. In some aspects, a poorly ionizing analytes such as a biomolecule will have a larger incident angle while better ionizing analytes such as low molecular weight biomolecules and organic compounds have smaller incident angle. Without wishing to be bound by any theory, it is believed that the differences in the incident angle results from the two different ionization mechanisms for each type of molecule. The poorly ionizing biomacromolecules may be desorbed by the droplet where multiple charges in the droplet may be transferred to the biomacromolecule. On the other hand, low molecular weight molecules may undergo charge transfer as either a proton or an electron. This charge transfer may be from a solvent ion to an analyte on the surface, from a gas phase solvent ion to an analyte on the surface, or from a gas phase solvent ion to a gas phase analyte molecule.

Additionally, the collection efficiency or the amount of desorbed analyte collected by the collector can be optimized by varying the collection distance from the inlet of the mass spectrometer and the surface as well as varying the collection angle (β). In general, the collection distance is relatively short from about 0 mm to about 5 mm. In some cases, the collection distance may be from about 0 mm to about 2 mm. Additionally, the collection angle (β) is also relatively small from about 1° to about 30° such as from 5° to 10°.

Each of these components may be individually adjusted to obtain an sufficient ionization and collection efficiencies. Within the DESI source, the sample may be placed on a 3D moving stage which allows precise and individual control over the ionization distance, the collection distance, the incident angle, and the collection angle.

The mass spectrometer may use a variety of different mass analyzers. Some non-limiting examples of different mass analyzers include time-of-flight, quadrupole mass filter, ion trap such as a 3D quadrupole ion trap, cylindrical ion trap, linear quadrupole ion trap, or an orbitrap, or a fourier transform ion cyclotron resonance device.

In some aspects, the present invention also provides methods for analyzing mass spectrometry data to determine the sample type and disease state. These methods include statistical methods, such as the least absolute shrinkage and selection operator (LASSO) method. The LASSO method can be applied to determine the presence of particular lipids, the presence of which may be indicative of sample type or disease state. This information can be used to make intrasurgical or preoperative decisions regarding treatment.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Determination of Tissue Type by MasSpec Pen

*Materials and methods.* Human tissue samples including thyroid, thymic, parathyroid, and lymph node samples were obtained from Baylor College of Medicine and the Cooperative Human Tissue Network. Samples were stored at -80°C until analysis, when they were thawed and analyzed at room temperature. Samples were analyzed using a Q Exactive mass spectrometer (Thermo Scientific) coupled to the MasSpec Pen. Analyzed regions of the tissue were annotated, and then the sample was frozen and sectioned at a thickness of 10µm. Tissue sections were H&E stained and evaluated by a pathologist. The least absolute shrinkage and selection operator (lasso) statistical method was used to identify molecular signatures and build a classification model for prediction.

*MasSpec Pen Analysis of Dissected Tissues.* The MasSpec Pen was used to analyze different tissue types such as parathyroid, normal thyroid, and lymph node tissue samples in the negative ion mode. The MasSpec Pen allowed for the detection of various diagnostic small molecules, including metabolites, fatty acids, and many glycerophospholipid species (GP), such as glycerophosphoinositols, glycerophosphoserines, and glycerophosphoethanolamines (PE). Although complex, the mass spectra obtained presented trends characteristic of parathyroid, normal thyroid, and normal lymph node tissues. For example, *m*/*z* 766.544, which is tentatively attributed to PE 38:4, was observed at a high relative abundance in parathyroid tissue while an unidentified species at *m*/*z* 822.478 was observed at a high relative abundance in normal thyroid tissue. For each sample analyzed, three mass spectra were averaged and used for data analysis. First pass issue determination for various tissue types is shown in Table 1.

**Table 1: Cervical Tissue Typing (Parathyroid versus Thyroid versus Lymph Node)**

| Predict | | | |
|---|---|---|---|
| True | Thyroid | Lymph | Parathyroid |
| Thyroid | 30 | 0 | 2 |
| Lymph | 1 | 16 | 3 |
| Parathyroid | 0 | 0 | 47 |

| | | | |
|---|---|---|---|
| Thyroid Accuracy: 93.75% Lymph Accuracy: 80.00% Parathyroid Accuracy: 100.00% Overall Accuracy: 93.9% | | | |

LASSO was then used to generate a statistical classifier and identify molecular signatures of parathyroid, normal thyroid, and normal lymph node tissues (Table 2A). Threefourths of the data was used to generate a model using LASSO and cross validation with an accuracy for tissue identification. An overall prediction accuracy of 100% on the withheld data was achieved, as seen in Table 2B, indicating that DESI-MS imaging and statistical prediction using LASSO is a promising way to identify tissues.

**Table 2A: Molecular signatures**

| Features: | | | |
|---|---|---|---|
| | Thyroid | Lymph | Parathyroid |
| m/z | 3.299751e-01 | -6.695472e-02 | -2.630203e-01 |
| 146.04 | 0.000000e+00 | 5.422916e-07 | 0.000000e+00 |
| 146.05 | 0.000000e+00 | 1.067104e-06 | 0.000000e+00 |
| 146.96 | 0.000000e+00 | 0.000000e+00 | 1.221110e-07 |
| 175.02 | 0.000000e+00 | -5.232571e-08 | 1.811669e-08 |
| 187.04 | 0.000000e+00 | 1.363333e-07 | 0.000000e+00 |
| 191.02 | 7.382345e-07 | 0.000000e+00 | 0.000000e+00 |
| 195.05 | 0.000000e+00 | 2.110800e-07 | 0.000000e+00 |
| 214.05 | 0.000000e+00 | 0.000000e+00 | 4.578956e-06 |
| 341.27 | 1.883022e-07 | 0.000000e+00 | 0.000000e+00 |
| 766.54 | 0.000000e+00 | 0.000000e+00 | 2.177878e-07 |
| 822.47 | 1.771216e-06 | 0.000000e+00 | 0.000000e+00 |
| 822.48 | 8.545613e-07 | 0.000000e+00 | 0.000000e+00 |
| 885.55 | -5.901954e-07 | 0.000000e+00 | 0.000000e+00 |

**Table 2B: Test Results**

| Predict | | | |
|---|---|---|---|
| True | Thyroid | Lymph | Parathyroid |
| Thyroid | 11 | 0 | 0 |
| Lymph | 0 | 6 | 0 |
| Parathyroid | 0 | 0 | 16 |

| | | | |
|---|---|---|---|
| Thyroid Accuracy: 100.0000% Lymph Accuracy: 100.0000% Parathyroid Accuracy: 100.0000% Overall Accuracy: 100.0000% | | | |

The MasSpec Pen was able to differentiate between parathyroid, normal thyroid, and normal lymph node tissues with high accuracies, indicating that this could be a valuable tool for rapid, non-destructive diagnosis of cervical tissues intraoperatively.

### Example 2 - Assessment of Thyroid Tissues and Fine Needle Aspirates by DESI-MS and LASSO

Fine Needle Aspirate biopsy is well-established for diagnosis of suspicious thyroid lesions. However, histologic discrimination between malignant follicular thyroid carcinomas (FTC) and benign follicular thyroid adenomas (FTA) is unachievable due to their similar cytological features. This results in a number of patients undergoing surgery for final diagnosis, and in the majority of these cases the lesion is found to be benign, thus resulting in an unnecessary surger. New technologies which provide accurate preoperative diagnosis of these lesions are greatly needed.

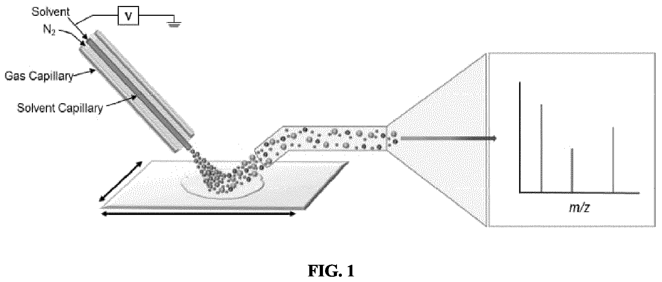

To address the need for accurate preoperative diagnosis, the molecular profiles of FTA, FTC, and normal thyroid were investigated using desorption electrospray ionization mass spectroscopy (DESI-MS) imaging, and the least absolute shrinkage and selection operator (LASSO) statistical method to characterize their molecular profiles (FIG. 1).

*Tissue preparation* - Human thyroid tissue samples were obtained from the Cooperative Human Tissue Network, Baylor College of Medicine, and the Kolling Institute of Medical Research Tumour Bank. 52 FTA, 25 FTC, and 37 normal thyroid samples were obtained. These samples were sectioned at a thickness of 16 µm and stored at -80°C until analysis.

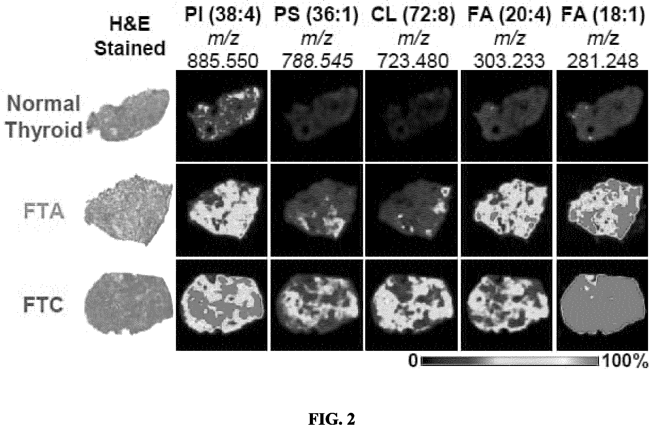

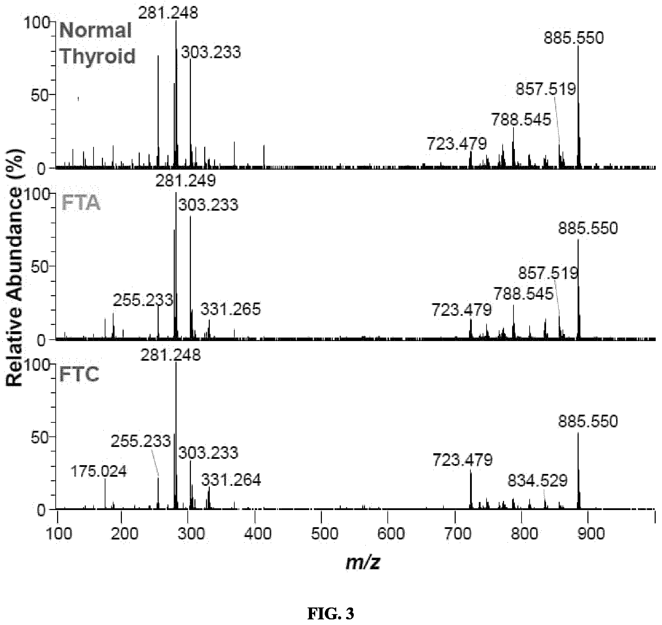

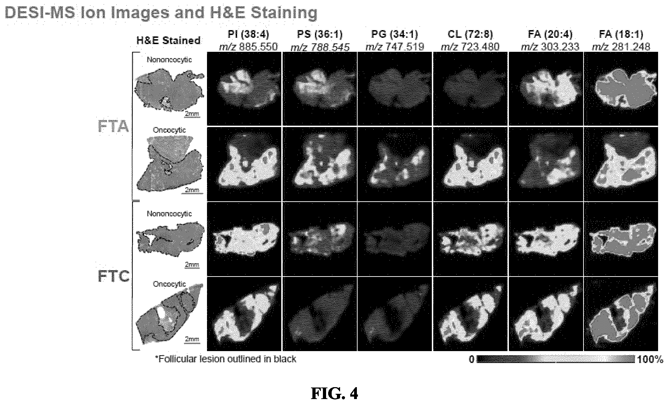

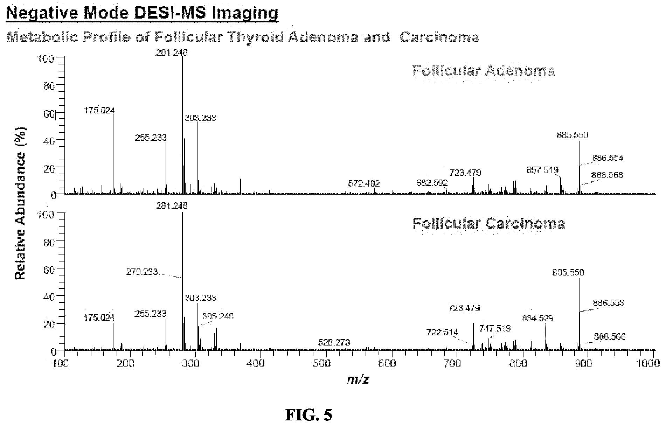

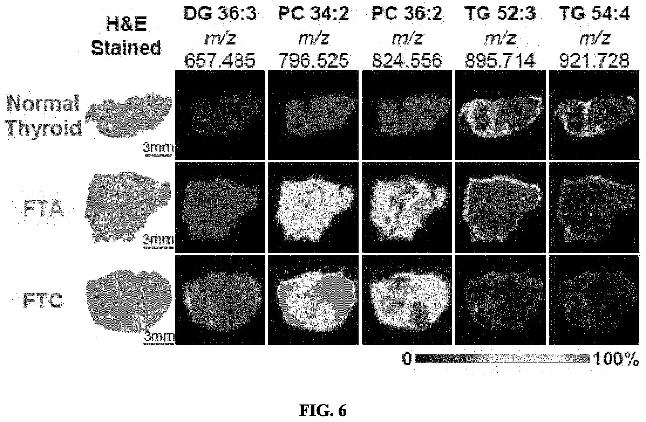

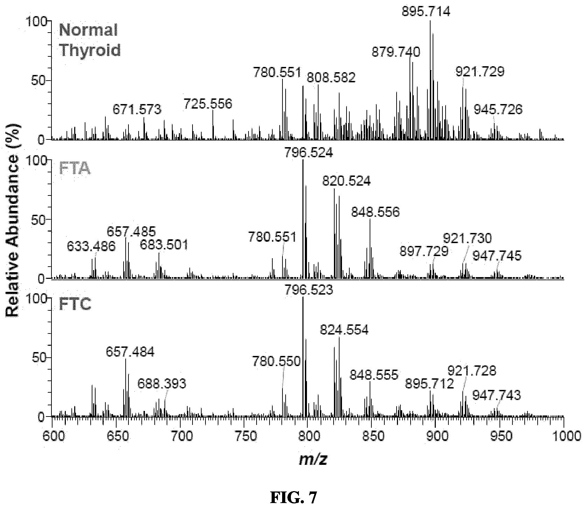

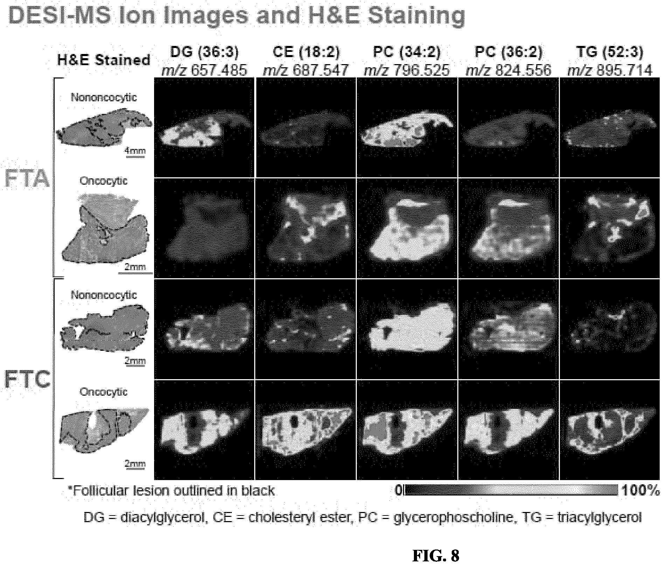

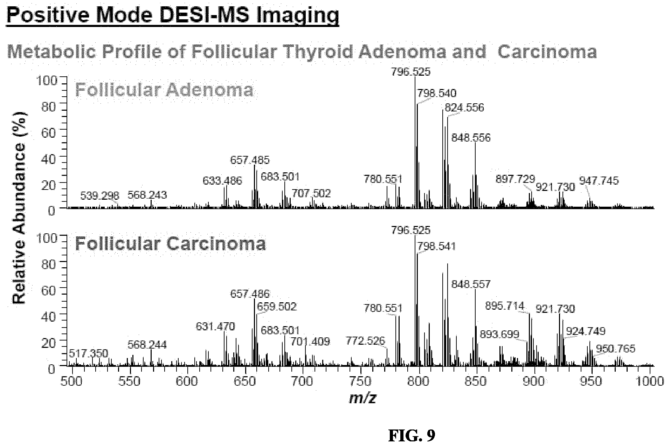

*DESI-MS imaging -* tissue sections were analyzed using a Q Exactive mass spectrometer (Thermo Fisher Scientific) fitted with a 2D Omni spray stage (Prosolia Inc.). DESI-MS imaging was performed at a spatial resolution of 200 um with a mass range of *m*/*z* 100-1500 in both the negative and positive ion modes. For negative ion mode, a solvent of dimethylformamide:acetonitrile (DMF:ACN) 1:1 (v/v) with 10 ppb sodium taurodeoxycholate hydrate as a lockmass compound was used at a flow rate of 1.2 µL/min. Pure ACN was used in positive ion mode at a flow rate of 3 µL/min. Representative images of tissue sections imaged using Negative Ion Mode DESI-MS imaging are shown in FIGS. 2 and 4, while their MS spectra are shown in FIGS. 3 and 5, respectively. Representative images of tissue sections imaged using Positive Ion Mode are shown in FIGS. 6 and 8, while the corresponding MS spectra are shown in FIGS. 7 and 9, respectively. Ion images were assembled using BioMap software.

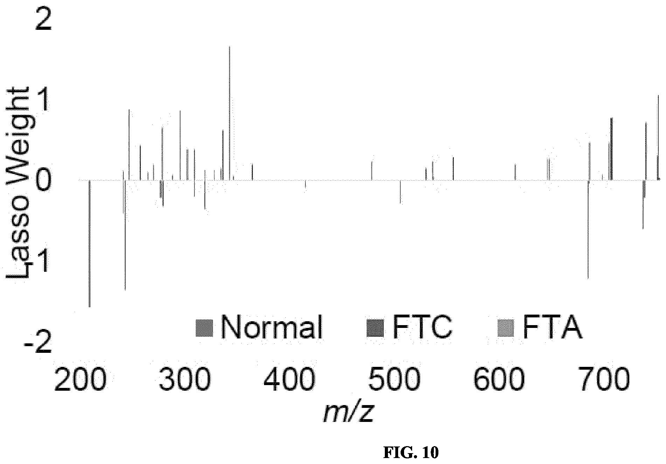

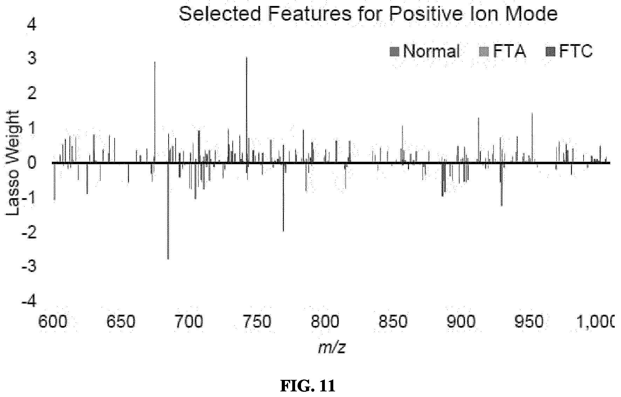

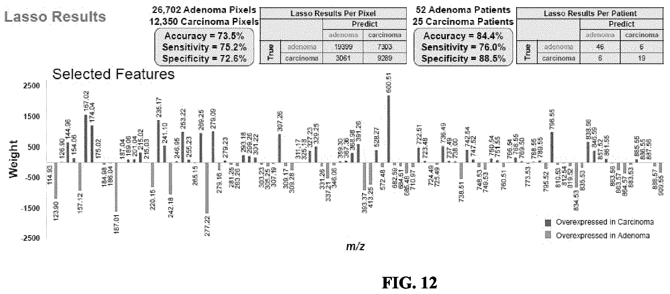

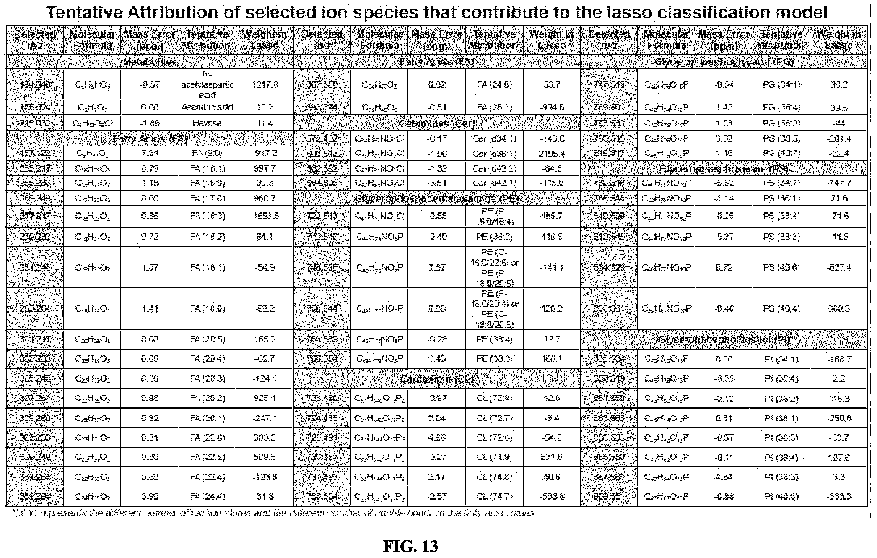

*Histopathology and Statistical Analysis* - After DESI-MS imaging, tissue sections were H&E stained and evaluated by light microscopy. Spectra corresponding to regions of pure FTA, FTC, and normal thyroid were selected and converted to text files to use for statistical analysis using MSiReader Software. LASSO prediction was performed to select a sparse set of features that can be used to discriminate between normal thyroid, FTA and FTC tissues. Lasso weights are depicted for selected *m*/*z* features for negative ion mode classification in FIGS. 10 and 12 (corresponding to the spectra in FIGS. 3 and 5, respectively), and positive ion mode in FIG. 11. The selected features were tentatively identified using high mass accuracy identifying various lipid species, including glycerolipids, sphingolipids, and glycerophospholipids. Tentative attribution of selected ion species contributing to the LASSO prediction can be seen in FIG. 13. The LASSO prediction results for the spectra depicted in FIGS. 3 and 7 are shown in Tables 1 and 2, while the LASSO prediction results for the spectra depicted in FIG. 5 can be seen at the top of FIG. 12. LASSO prediction using negative ion MS as shown in FIG. 3 had an 80.9% agreement with the pathologist's assessment, while positive ion MS as shown in FIG. 7 had a 94.4% agreement with the pathologist's diagnosis.

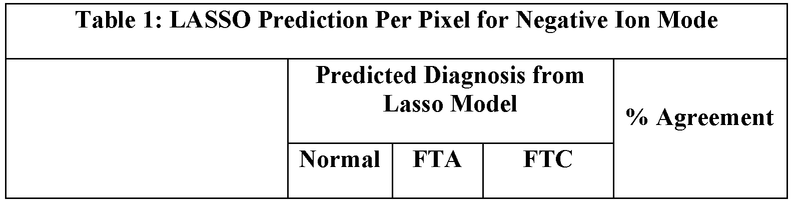

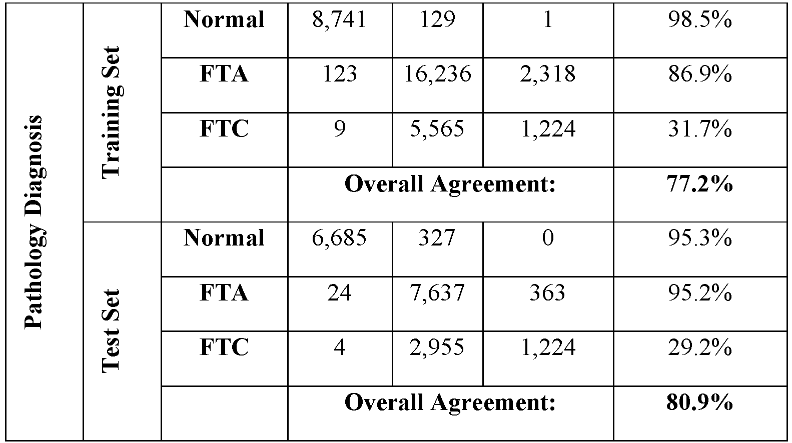

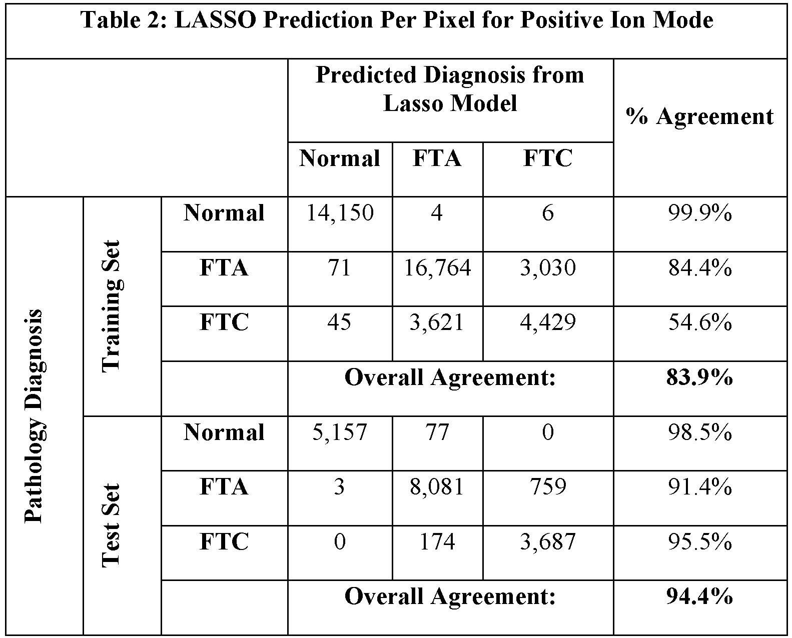

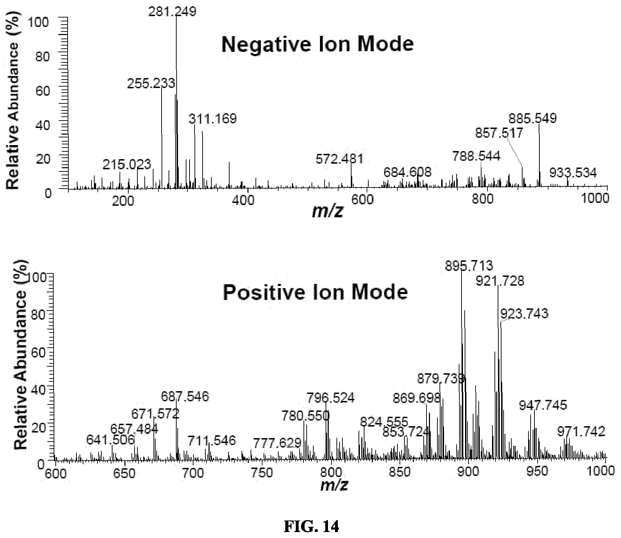

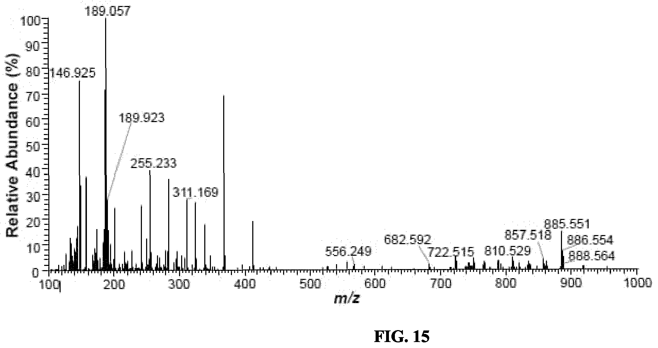

*Fine Needle Aspiration Analysis* - Fine Needle Aspirates (FNA) are regularly taken to biopsy tissues before surgery, and could be a powerful tool for determining the need for surgery. To determine whether the MasSpec pen and DESI-MS could be used to determine the status of a tissue, fine needle aspiration samples were collected from clinical practice at Baylor College of Medicine. Preliminary analysis of 12 FNA samples was performed using DESI-MS imaging in positive and negative ion modes as above. Preliminary analysis yielded the mass spectra pictured in FIGS. 14 and 15, indicating that rich molecular information can be obtained from FNA samples. This indicates that DESI-MS of FNA samples may be extremely valuable for preoperative diagnosis of thyroid lesions.

### Example 3 - Preoperative Diagnosis of Thyroid Nodules using Mass Spectrometry Imaging of Fine Needle Aspiration Biopsies

### DESI-MS Imaging of Thyroid Tissues

DESI-MS imaging was performed in the negative and positive ion modes on 178 banked human thyroid tissue samples, including 37 normal thyroid, 71 follicular thyroid adenoma (FTA), 33 follicular thyroid carcinoma (FTC), and 37 papillary thyroid carcinoma (PTC) FTA and FTC classes comprised of both oncocytic and non-oncocytic samples). In the negative ion mode, DESI analysis allowed for detection and characterization of many glycerophospholipid (GP) species, sphingolipids, fatty acids, and small metabolites while the positive ion mode allowed for the detection and characterization of phosphatidylcholine, diacylglycerol, and triacylglycerol species. Although complex, the mass spectra profiles obtained presented trends in ion abundances that were characteristic of PTC, FTC, FTA, and normal thyroid tissues. The negative ion mode had superior performance for the discrimination of thyroid tissue types and will be the focus of this manuscript, see supporting information for positive ion mode results.

### Development of Statistical Models

After DESI-MS analysis, the analyzed tissue sections were hematoxylin and eosin (H&E) stained and evaluated by an expert pathologist to determine regions of clear diagnosis. Spectra corresponding to regions of pure PTC, FTC, FTA, and normal thyroid were extracted and used to build statistical models to use for prediction on FNA samples. For statistical analysis, the normal thyroid and FTA classes were combined to create a benign thyroid class because the greatest clinical relevance lies in discriminating thyroid cancer from benign thyroid tissue. The least absolute shrinkage and selection operation (lasso) statistical method was used to build two separate classification models to use for prediction: PTC vs. benign thyroid and FTC vs. benign thyroid. The lasso method selects a sparse set of m/z features that are able to discriminate each tissue class and assigns them a mathematical weight. This group of selected features, along with their lasso weights, is used as a classification model that can predict disease status of tissues. For each model, the data was randomly split into a training set and a validation set, on a per sample basis. The training set of data was used to generate a model using lasso along with cross validation (CV) while the performance of the model was assessed by predicting on the withheld validation set. The prediction accuracies of training and test sets were calculated for each model and are presented as % agreement with the diagnosis given by pathology.

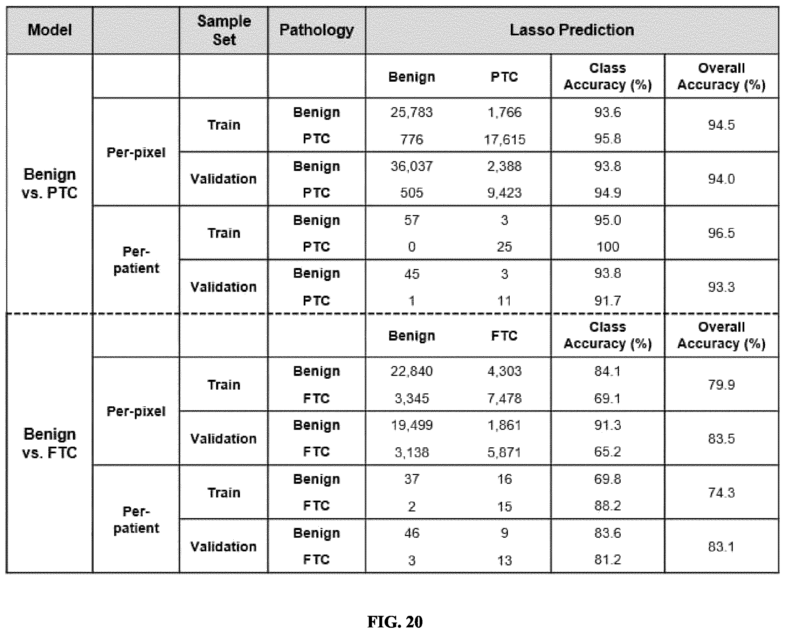

First, a predictive model discriminating PTC from benign thyroid was built with lasso along with CV using 45,940 pixels of data from 85 tissue samples. For this model, a total of 67 m/z features were selected to discriminate PTC from benign thyroid with an overall CV prediction accuracy of 94.5%, shown in the table of FIG. 20. The selected m/z features were tentatively identified using high mass accuracy and tandem MS as a variety of biological species such as ceramides and GP species, including cardiolipins (CL), phosphatidylethanolamines (PE), and phosphatidylinositols (PI). When predicting on the withheld validation set of data (48,353 pixels, 60 samples), a 94.0% agreement with pathology was achieved, with a sensitivity of 94.9% and specificity of 93.8%. The high prediction performance of this model on a validation set of data demonstrate the potential for lipid and metabolite information to discriminate thyroid tumor types.

While high performance was achieved for the PTC vs. benign thyroid model, the greater clinical challenge lies in differentiating benign thyroid, such as FTA, from FTC, as these can be indistinguishable from FNA. This motivated a second lasso classifier: benign thyroid vs. FTC. This was particularly challenging as the spectra obtained from FTA and FTC samples appeared to be qualitatively similar. Using a training set of 37,966 pixels from 70 patients, lasso was used to build a model with an overall CV agreement with pathology of 79.9%, as shown in the table of FIG. 20. The lasso selected a total of 772 m/z features for discriminating benign thyroid from FTC. This model was then used to predict on the withheld validation set of data (30,369 pixels, 71 samples) and achieved an overall per-pixel agreement with pathology of 74.3%, with a sensitivity of 65.2% and specificity of 91.3%. The per-patient resultsreveal that only 3 FTC samples were misclassified as benign in the validation set of samples, resulting in a per-patient sensitivity of 81.2%.

The highest weight selected m/z features for the benign vs. FTC model were tentatively identified using high mass accuracy and included a variety of fatty acids (FA), small metabolites, ceramides, and GP species. Among the ions with the highest weights for characterizing benign thyroid tissue were FA 20:4 (m/z 303.233), phosphatidylserine (PS) 36:1 (m/z 788.546), and phophatidylglycerol (PG) 32:1 (m/z 719.490). Among the ions with the highest weights for characterizing FTC were PI 38:4 (m/z 885.550), Cer d36:1 (m/z 600.513), and PE 36:1 (m/z 744.554).

### DESI-MS Analysis and Lasso Prediction on Clinical FNA Samples

Once prediction models were built based on tissue imaging data, the predictive performance of these models on FNA samples was assessed. To achieve this, FNA samples were prospectively collected at Baylor College of Medicine for DESI-MS analysis. Samples were collected during clinical practice from patients undergoing routine FNA biopsy for thyroid nodule diagnosis. Additionally, to increase our sample size, FNAs were collected from patients undergoing surgical removal of thyroid nodules. In these cases, FNAs were collected *in vivo* prior to surgery as well as on the ex vivo thyroid nodules. For samples collected during routine FNA biopsy, the sample diagnosis was determined by routine pathological evaluation of a second FNA pass collected concurrently from the same patient. For samples collected during surgery, the sample diagnosis was determined from the final pathology of the surgically removed thyroid nodule.

After FNA collection, samples were deposited on a glass slide, frozen, and stored at -80°C until analysis. In the current clinical FNA smears collected, the distribution of the thyroid cells is not uniform throughout the FNA smear; therefore DESI-MS imaging is performed on the whole sample area in the first the positive and then the negative ion mode. A total of 87 FNA smears from 63 patients were analyzed using DESI-MS imaging. These analyzed samples comprised of 20 benign, 15 FTA, 1 FTC, 25 PTC, 1 indeterminate, and 1 non-diagnostic FNA sample. The profiles of the FNAs analyzed show that hundreds of peaks corresponding to various lipids and metabolites are detected, indicating that rich molecular information can be obtained from FNA even though FNA samples have a lower cell density than tissue samples. After DESI-MS analysis, all FNA samples were hematoxylin and eosin stained and brought to an expert pathologist for evaluation. Regions corresponding to follicular thyroid cells were identified by an expert and then the mass spectra corresponding to thyroid cells were extracted using MSiReader software. After evaluation by the expert, there were 17 benign, 17 FTA, 1 FTC, and 30 PTC FNA smears from 51 patients (some patients had multiple FNA smears) that had regions of follicular thyroid cells from which data could be extracted.

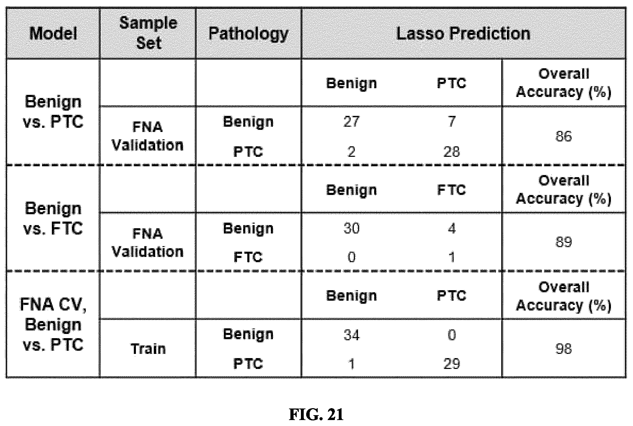

Once the FNA samples had been analyzed and extracted the mass spectra corresponding to follicular thyroid cells, the performance of the lasso classifiers that were built from tissue imaging data on the FNA samples were tested. The benign vs. PTC model was used to predict on the 17 benign, 17 FTA, and 30 PTC FNA samples, with the results shown in the table of FIG. 21. From the negative mode data, the PTC vs. benign thyroid model yielded an overall per-sample accuracy of 85.9%, with a benign accuracy of 79.4% and a PTC accuracy of 93.3%. Furthermore, the FTC vs. benign thyroid model was used to predict on the benign, FTA, and FTC FNA biopsies with an overall per sample accuracy of 89%, which is extremely promising considering FTA and FTC are indistinguishable using histopathology of FNA smears.

While the tissue imaging classifiers performed well when predicting on spectra from clinical FNA samples, test were performed to potentially further improve prediction results by building a classifier from the FNA data directly. A benign thyroid vs. PTC lasso model was built from the clinical FNA data using the entire data set as a training set (64 samples), with the CV predictions shown in the table of FIG. 21. Using CV, an overall agreement with pathology of 98% was achieved with only one misclassified PTC sample. This further supports that the metabolic information obtained from FNA samples is predictive of disease status, indicating that this method has the potential to be a valuable tool for preoperative diagnosis of thyroid lesions.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

The following aspects are preferred embodiments of the invention.
1. An assay method comprising:
   (a) obtaining a fine-needle aspirate biopsy sample from a subject; and
   (b) performing mass spectrometry (MS) on the sample.
2. The method of aspect 1, wherein the fine-needle aspirate biopsy is a thyroid biopsy from the subject.
3. The method of aspect 1, further comprising performing a fine-needle aspirate biopsy on the subject.
4. The method of aspect 1, further comprising performing an ultra sound guided fine-needle aspirate biopsy of a thyroid nodule on the subject.
5. The method of aspect 1, further defined an *ex vivo* method.
6. The method of aspect 1, further defined as a method for detecting if a thyroid nodule from which the fine-needle aspirate biopsy was obtained is benign or malignant.
7. The method of aspect 1, further defined as a method for detecting cancer in the subject.
8. The method of aspect 1, further defined as a method for detecting thyroid cancer in the subject.
9. The method of aspect 1, further comprising identifying the sample as associated with cancer cells based on the MS analysis.
10. The method of aspect 9, wherein said identifying comprises detecting one or more of the molecules in FIG. 13.
11. The method of aspect 6, wherein the thyroid cancer is oncocytic thyroid cancer.
12. The method of aspect 6, wherein the thyroid cancer is follicular thyroid carcinoma.
13. The method of aspect 6, wherein the thyroid nodule is benign tissue.
14. The method of aspect 13, wherein the benign tissue is a thyroid adenoma or normal thyroid tissue.
15. The method of aspect 6, wherein the thyroid cancer is medullary thyroid cancer.
16. The method of aspect 6, wherein the thyroid cancer is anaplastic thyroid cancer.
17. The method of aspect 6, wherein the thyroid cancer is papillary thyroid cancer, follicular thyroid cancer, anaplastic thyroid cancer, or medullary thyroid cancer.
18. The method of aspects 1, wherein the ambient ionization MS comprises DESI-MS imaging.
19. The method of aspects 1, wherein the fine-needle aspirate biopsy sample is expelled onto a glass slide, and the glass slide directly imaged by DESI-MS in the region with FNA material.
20. The method of aspect 1 or 5, wherein performing ambient ionization comprises measuring a level of a lipids and/or metabolites in the sample.
21. The method of aspect 6, further comprising obtaining a reference profile and detecting the presence of cancer cells by comparing the profile from the sample to a reference profile.
22. The method of aspect 21, wherein the reference profile is obtained from the same subject.
23. The method of aspect 21, wherein the reference profile is obtained from a different subject or a combination of different subjects.
24. The method of aspect 1, further comprising:
   (c) administering at least a first anticancer therapy to a subject identified to have a cancer.
25. The method of aspect 24, wherein the cancer is a thyroid cancer.
26. The method of aspect 24, wherein the anticancer therapy comprises radiation, immunotherapy, surgery or chemotherapy therapy.
27. The method of aspect 25, wherein the cancer is a thyroid cancer and the anticancer therapy is an iodine based therapy.
28. The method of aspect 6, wherein a statistical classifier is built from the lipid and/or metabolic information obtained from reference profiles by MS methods to identify if a thyroid sample obtained from a nodule is benign or malignant.
29. The method of aspect 28, wherein the statistical classifier is built from reference profiles obtained from tissue samples by MS methods.
30. The method of aspect 28, wherein the statistical classifier is built from reference profiles obtained from fine-needle biopsy samples by MS methods.
31. The method of aspect 28, wherein the statistical classifier is built from reference profiles obtained from a combination of tissue samples and fine-needle biopsy samples by MS methods.
32. The method of aspect 28, wherein the statistical classifier is built as a two-class classifier that can identify nodules as benign thyroid adenomas or malignant thyroid carcinomas.
33. The method of aspect 28, wherein the statistical classifier is built as a two-class classifier that can identify nodules as benign thyroid, including adenomas and normal thyroid, or malignant thyroid carcinomas.
34. The method of aspect 28, wherein the statistical classifier is built as a three-class classifier that can identify nodules as benign thyroid, thyroid adenomas, or thyroid carcinomas.
35. The method of aspect 28, wherein the thyroid tumor is follicular neoplasm.
36. The method of aspect 28, wherein the thyroid tumor is papillary neoplasm.
37. The method of aspect 28, wherein the thyroid tumor is medullary neoplasm.
38. The method of aspect 28, where the classifier predicts on the data acquired from one or more pixels of the FNA in the glass slide.
39. The method of aspect 28, where the prediction is performed on one or more pixels that contain one or more cells.
40. The method of aspect 28, where the FNA classification result is based on a single pixel result or a combination of the predictions given to each individual pixel.
41. The method of aspect 29, where the cutoff value for sample classification is optimized based on results for FNA samples.
42. The method of aspect 6, where the FNA sample is subjected to histology after MS analysis.
43. The method of aspect 28, where one or more pixels with one or more cells are identified in the FNA sample by an auxiliary technique before or after MS analysis.
44. The method of aspect 28, where the auxiliary technique is pathology.
45. The method of aspect 28, where the auxiliary technique is a spectroscopic method.
46. The method of aspect 28, where the MS result is combined to genetic classifier result to obtain a final diagnosis for the FNA analyzed.
47. The method of aspect 6, wherein MS prediction is made when cell counts are sufficient.
48. The method of aspect 6, wherein a pixel prediction is made when reaching probability cut-off of a pixel.
49. The method of aspect 6, wherein prediction is made when reaching a pixel cut-off from one FNA sample.
50. A method for assessing tissue samples from a subject during cervical surgery comprising:
   (a) applying a fixed or discrete volume of a solvent to a tissue site in the subject;
   (b) collecting the applied solvent to obtain a liquid sample;
   (c) subjecting the sample to mass spectrometry analysis; and
   (d) identifying the tissue site as thyroid tissue, thymic tissue, parathyroid tissue or lymph node tissue.
51. The method of aspect 50, wherein the method is automated.
52. The method of aspect 50, further comprising imaging the tissue site before said surgery.
53. The method of aspect 52, wherein the imaging comprises a CT or MRI scan.
54. The method of aspect 52, wherein the imaging is used to produce a 3-dimension representation of the surgical site.
55. The method of aspect 50, wherein said identifying is completed by a computer.
56. The method of aspect 50, further comprising surgically removing a portion of tissue based on said identifying.
57. The method of aspect 56, wherein the surgical removal is automated.
58. The method of aspect 57, wherein the identifying is completed by a computer and used to guide the surgical removal of a selected tissue.
59. The method of aspect 58, wherein the computer comprises imaging data from the tissue site that is used to guide the surgery.
60. The method of aspect 56, wherein the portion of tissue removed is thyroid tissue.
61. The method of aspect 50, wherein said identifying comprises detecting one or more of the molecules in Table 2.
62. The method of aspect 50, further comprising identifying the tissue site as cancer or non-cancer tissue.
63. The method of aspect 50, wherein the fixed or discrete volume of a solvent is not applied as a spray.
64. The method of aspect 50, wherein the fixed or discrete volume of a solvent is applied as a droplet.
65. The method of aspect 50, wherein the fixed or discrete volume of a solvent is applied at using a pressure of less than 100 psig.
66. The method of aspect 50, wherein the fixed or discrete volume of a solvent is applied at using a pressure of less than 10 psig.
67. The method of aspect 50, wherein the fixed or discrete volume of a solvent is applied at using a mechanical pump to move the solvent through a solvent conduit.
68. The method of aspect 50, wherein collecting the applied solvent comprises applying a negative pressure to pull the sample into a collection conduit and/or applying a gas pressure to push the sample into a collection conduit.
69. The method of aspect 50, wherein collecting the applied solvent comprises applying a negative pressure to pull the sample into a collection conduit and applying a positive pressure to push the sample into a collection conduit.
70. The method of aspect 68, wherein the solvent is applied through a solvent conduit that is separate from the collection conduit.
71. The method of aspect 70, wherein the gas pressure is applied through a gas conduit that is separate from the solvent conduit and the collection conduit.
72. The method of aspect 68, wherein applying a gas pressure to push the sample into a collection conduit comprises applying a pressure of less than 100 psig.
73. The method of aspect 50, wherein the method produces no detectable physical damage to the tissue site.
74. The method of aspect 50, wherein the method does not involve application of ultrasonic or vibrational energy to the tissue.
75. The method of aspect 50, wherein the solvent is sterile.
76. The method of aspect 50, wherein the solvent is pharmaceutically acceptable formulation.
77. The method of aspect 76, wherein the solvent is an aqueous solution.
78. The method of aspect 77, wherein the solvent is sterile water.
79. The method of aspect 77, wherein the solvent consists essentially of water.
80. The method of aspect 77, wherein the solvent comprises from about 1 to 20% of an alcohol.
81. The method of aspect 80, wherein the alcohol comprises ethanol.
82. The method of aspect 50, wherein the discrete volume of solvent is between about 0.1 and 100 µL.
83. The method of aspect 82, wherein the discrete volume of solvent is between about 1 and 50 µL.
84. The method of aspect 50, wherein collecting the applied solvent is between 0.1 and 30 seconds after the applying step.
85. The method of aspect 84, wherein collecting the applied solvent is between 1 and 10 seconds after the applying step.
86. The method of aspect 50, wherein the tissue site is in living tissue that is being surgically assessed.
87. The method of aspect 50, further comprising collecting a plurality liquid samples from a plurality of tissue sites.
88. The method of aspect 87, wherein the liquid samples are collected with a probe.
89. The method of aspect 88, wherein the probe is washed between collection of the different samples.
90. The method of aspect 88, wherein the probe comprises a disposable portion that contacts the tissue site and where said disposable portion is changed between collection of the different samples.
91. The method of aspect 88 wherein the probe comprises a collection tip and further comprising ejecting the collection tip from the probe after the liquid samples are collected.
92. The method of aspect 87, wherein the plurality of tissue sites comprises 2, 3, 4, 5, 6, 7, 8, 9 or 10 tissues sites.
93. The method of aspect 92, wherein the plurality of tissue sites surrounds a section of tissue that has been surgically resected.
94. The method of aspect 93, wherein the resected tissue is a tumor.
95. The method of aspect 50, further defined as an intraoperative method.
96. The method of aspect 50, wherein the mass spectrometry comprises ambient ionization MS.
97. The method of aspect 50, wherein subjecting the sample to mass spectrometry analysis comprises determining a profile corresponding to the tissue site.
98. The method of aspect 97, further comprising comparing the profile to a reference profile to identify tissue sites that include diseased tissue.
99. The method of aspect 98, further comprising resecting tissue sites that are identified to include diseased tissue.

### REFERENCES

The following references, to the extent that they provide exemplary procedural or other details supplementary to those set forth herein, are specifically incorporated herein by reference.
U.S. Patent Application No. 15/648,276
U.S. Patent Application No. 15/692,167
Zhang et al., "Nondestructive tissue analysis for ex vivo and in vivo cancer diagnosis using a handheld mass spectrometry system." Science translational medicine 9.406, 2017

## Claims

1. A method for assessing tissue samples using mass spectrometry, the method comprising:
(a) applying a fixed or discrete volume of a solvent to a tissue site;
(b) collecting the applied solvent to obtain a liquid sample;
(c) subjecting the sample to mass spectrometry analysis; and
(d) identifying the tissue site as thyroid tissue, thymic tissue, parathyroid tissue or lymph node tissue.

2. The method of claim 1, wherein said identifying comprises detecting one or more of the molecules in Table 2.

3. The method of claim 1,
wherein the tissues samples are cervical tissue samples; and/or
wherein the method further comprises identifying the tissue site as cancer or non-cancer tissue.

4. The method of claim 1, wherein the fixed or discrete volume of a solvent is not applied as a spray, preferably wherein the fixed or discrete volume of a solvent is applied as a droplet.

5. The method of claim 1, wherein the fixed or discrete volume of a solvent is applied at using a pressure of less than 100 psig.

6. The method of claim 1, wherein the fixed or discrete volume of a solvent is applied using a mechanical pump to move the solvent through a solvent conduit.

7. The method of claim 1, wherein collecting the applied solvent comprises applying a negative pressure to pull the sample into a collection conduit and/or applying a gas pressure to push the sample into a collection conduit.

8. The method of claim 1, wherein the method produces no detectable physical damage to the tissue site.

9. The method of claim 1, wherein the method does not involve application of ultrasonic or vibrational energy to the tissue.

10. The method of claim 1, wherein the discrete volume of solvent is between about 0.1 and 100 µL.

11. The method of claim 1, wherein the applied solvent is collected between 0.1 and 30 seconds after the applying step.

12. The method of claim 1, further comprising collecting a plurality liquid samples from a plurality of tissue sites, preferably wherein the plurality of liquid samples are collected with a probe, and wherein:
the probe is washed between collection of the plurality of samples;
the probe comprises a disposable portion that contacts the tissue site and where said disposable portion is changed between collection of the different samples; or
the probe comprises a collection tip and the method further comprises ejecting the collection tip from the probe after the liquid samples are collected.

13. The method of claim 1, wherein the mass spectrometry comprises ambient ionization MS.

14. The method of claim 1, wherein subjecting the sample to mass spectrometry analysis comprises determining a profile corresponding to the tissue site.

15. The method of claim 1, wherein subjecting the sample to mass spectrometry analysis comprises determining a profile corresponding to the tissue site, and wherein the method further comprises comparing the profile to a reference profile to identify tissue sites that include diseased tissue.
